# EUROPEAN PATENT APPLICATION

(11) **EP 4 524 979 A1**
(43) Date of publication of application: **19.03.2025**
(21) Application number: 23846048.9
(22) Date of filing: 14.06.2023
(51) Int. Cl.: G16H 20/00

(54) **COMPUTER PROGRAM, PAIN MANAGEMENT ASSISTANCE DEVICE, AND PAIN MANAGEMENT ASSISTANCE METHOD**

(30) Priority: 25.07.2022 JP 2022118142
(71) Applicant: Terumo Kabushiki Kaisha, Tokyo 151-0072 (JP)
(72) Inventor: SHIMADA, Naoya, Ashigarakami-gun, Kanagawa 259-0151 (JP); KIKUCHI, Hideka, Ashigarakami-gun, Kanagawa 259-0151 (JP); YAGO, Seiji, Ashigarakami-gun, Kanagawa 259-0151 (JP); MARUYAMA, Tomoji, Ashigarakami-gun, Kanagawa 259-0151 (JP); TAKEUCHI, Takanori, Tokyo 163-1450 (JP); YOKOUCHI, Atsushi, Tokyo 163-1450 (JP); FUJII, Naoko, Ashigarakami-gun, Kanagawa 259-0151 (JP)
(74) Representative: KIPA AB
(86) International application number: PCT/JP2023/022146
(87) International publication number: WO 2024/024314

(57) **Abstract**

There are provided a computer program, a pain management support device, and a pain management support method capable of supporting appropriate pain management.

A computer program causes a computer to execute a process of repeatedly obtaining pain-related information associated with pain of a patient at required timing, storing the obtained pain-related information, obtaining medical examination information associated with a medical examination of the patient, storing the obtained medical examination information, generating pain management support information on the basis of the pain-related information and the medical examination information, and outputting the generated pain management support information.

## Description

### Technical Field

The present invention relates to a computer program, a pain management support device, and a pain management support method.

### Background Art

Patent Literature 1 discloses a medical examination information management system including a plurality of treatment section servers that provides medical examination information stored in databases of a plurality of treatment sections in a medical institution, and a specified disease treatment integrated server that obtains specified disease medical examination information from the treatment section servers and provides integrated specified disease medical examination information from an integrated database, in which the medical examination information regarding the specified disease, such as cancer, in all therapies obtained by combining surgery, a radiation therapy, chemotherapy, and palliative care, and registration-related information for a wide range of specified diseases are made available for reference so that a progress status of treatment may be grasped with high immediacy.

### Citation List

### Patent Literature

Patent Literature 1: JP 2010-3135 A

### Summary of Invention

### Technical Problem

Since pain caused by a disease, such as cancer, is directly linked to quality of life (QOL) of a patient, appropriate pain management needs to be implemented. However, for example, when the patient under recuperation at home is examined by a doctor, it is difficult to exactly tell the doctor about the pain and various symptoms felt by the patient within the period from the previous examination. Furthermore, there is no general methodology by which the doctor extracts a condition of the patient and selects an appropriate analgesic method at the time of the examination. Accordingly, appropriate pain management is impeded.

The present invention has been conceived **in** view of such circumstances, and aims to provide a computer program, a pain management support device, and a pain management support method capable of supporting appropriate pain management.

### Solution to Problem

(1) A computer program according to the present invention causes a computer to execute a process of repeatedly obtaining pain-related information associated with pain of a patient at required timing, storing the obtained pain-related information, obtaining medical examination information associated with a medical examination of the patient, storing the obtained medical examination information, generating pain management support information on the basis of the pain-related information and the medical examination information, and outputting the generated pain management support information.
   Here, according to an embodiment of the present invention,
(2) In the computer program according to (1) described above, the pain-related information preferably includes at least one of an assessment index for assessing the pain, vital data, medication information, or a physical condition.
(3) In the computer program according to (1) or (2) described above, the medical examination information preferably includes at least one of diagnosis information, examination information, or prescription information.
(4) In the computer program according to any one of (1) to (3) described above, the pain management support information preferably includes at least one of a pain assessment result, recommended prescription information, or an instruction for a doctor visit for supporting the patient.
(5) In the computer program according to any one of (1) to (4) described above, the pain management support information preferably includes at least one of a pain assessment result, necessity of an interview, a medication result, recommended prescription information, or a recommended treatment method for supporting a doctor who examines the patient.
(6) The computer program according to any one of (1) to (5) described above preferably causes the computer to execute the process of outputting, to a patient terminal device or a support person terminal device, a pain assessment result indicating presence or absence of the pain of the patient on the basis of the pain-related information, and outputting recommended prescription information to the patient terminal device or the support person terminal device when the pain is present.
(7) The computer program according to (6) described above preferably causes the computer to execute the process of outputting, to the patient terminal device, a pain assessment result indicating an administration result before and after administration of a medicine based on the recommended prescription information, and outputting an instruction for a doctor visit to the patient terminal device on the basis of the administration result.
(8) The computer program according to any one of (1) to (7) described above preferably causes the computer to execute the process of outputting, to a doctor terminal device, a temporal change of a pain assessment result indicating an effect of an analgesic on the patient who is taking the analgesic, and outputting recommendation of a medication change or necessity of an interview to the doctor terminal device on the basis of the effect of the analgesic.
(9) The computer program according to any one of (1) to (8) described above preferably causes the computer to execute the process of outputting, to a doctor terminal device, a temporal change of a pain assessment result indicating an effect of an analgesic on the patient who is taking the analgesic, and outputting, to the doctor terminal device, a coping method, a recommended treatment method, or referral information regarding a doctor experienced in the recommended treatment method on the basis of the effect of the analgesic.
(10) The computer program according to any one of (1) to (9) described above preferably causes the computer to execute the process of outputting, to a doctor terminal device, a check item needed to select recommended prescription information or a recommended treatment method at a time of an examination, receiving input information for the check item, and outputting the recommended prescription information or the recommended treatment method to the doctor terminal device on the basis of the input information.
(11) The computer program according to any one of (1) to (10) described above preferably causes the computer to execute the process of outputting a question for evaluating a state of the pain of the patient to a patient terminal device, receiving an input of an answer to the question, and outputting a coping method to the patient terminal device on the basis of the answer.
(12) The computer program according to any one of (1) to (11) described above preferably causes the computer to execute the process of outputting a plurality of assumed questions related to the pain of the patient to a patient terminal device, and outputting, to the patient terminal device, an answer to an assumed question selected by the patient from among the plurality of assumed questions.
(13) The computer program according to any one of (1) to (12) described above preferably causes the computer to execute the process of outputting a check item needed to generate the pain management support information to a doctor terminal device, receiving input information for the check item, and generating the pain management support information on the basis of the input information.
(14) A pain management support device according to the present invention includes a first acquisition unit that repeatedly obtains pain-related information associated with pain of a patient at required timing, a first storage unit that stores the obtained pain-related information, a second acquisition unit that obtains medical examination information associated with a medical examination of the patient, a second storage unit that stores the obtained medical examination information, a generation unit that generates pain management support information on the basis of the pain-related information and the medical examination information, and an output unit that outputs the generated pain management support information.
(15) A pain management support method according to the present invention includes repeatedly obtaining pain-related information associated with pain of a patient at required timing, storing the obtained pain-related information, obtaining medical examination information associated with a medical examination of the patient, storing the obtained medical examination information, generating pain management support information on the basis of the pain-related information and the medical examination information, and outputting the generated pain management support information.

### Advantageous Effects of Invention

According to the present invention, appropriate pain management may be supported.

### Brief Description of Drawings

Fig. 1 is a diagram illustrating an exemplary configuration of a pain management support system according to the present embodiment.
Fig. 2 is a diagram illustrating an exemplary configuration of a pain management support device.
Fig. 3 is a diagram illustrating information collection by the pain management support device.
Fig. 4A is a diagram illustrating exemplary pain-related information.
Fig. 4B is a diagram illustrating exemplary medical examination information.
Fig. 5 is a diagram illustrating provision of pain management support information by the pain management support device.
Fig. 6A is a diagram illustrating exemplary pain management support information for a patient.
Fig. 6B is a diagram illustrating exemplary pain management support information for a doctor.
Fig. 7 is a diagram illustrating a method of generating pain management support information.
Fig. 8 is a diagram illustrating a first example of pain management support information on the patient side.
Fig. 9 is a diagram illustrating a second example of the pain management support information on the patient side.
Fig. 10 is a diagram illustrating a first example of pain management support information on the doctor side.
Fig. 11 is a diagram illustrating a second example of the pain management support information on the doctor side.
Fig. 12 is a diagram illustrating a third example of the pain management support information on the doctor side.
Fig. 13 is a diagram illustrating a fourth example of the pain management support information on the doctor side.
Fig. 14 is a diagram illustrating a fifth example of the pain management support information on the doctor side.
Fig. 15 is a diagram illustrating a sixth example of the pain management support information on the doctor side.
Fig. 16 is a diagram illustrating a seventh example of the pain management support information on the doctor side.
Fig. 17 is a diagram illustrating an eighth example of the pain management support information on the doctor side.
Fig. 18 is a diagram illustrating a ninth example of the pain management support information on the doctor side.
Fig. 19 is a diagram illustrating a tenth example of the pain management support information on the doctor side.
Fig. 20 is a diagram illustrating a third example of the pain management support information on the patient side.
Fig. 21 is a diagram illustrating a fourth example of the pain management support information on the patient side.
Fig. 22 is a diagram illustrating a fifth example of the pain management support information on the patient side.
Fig. 23 is a diagram illustrating a sixth example of the pain management support information on the patient side.
Fig. 24 is a diagram illustrating a seventh example of the pain management support information on the patient side.
Fig. 25 is a diagram illustrating an eighth example of the pain management support information on the patient side.
Fig. 26 is a diagram illustrating a ninth example of the pain management support information on the patient side.
Fig. 27 is a diagram illustrating an 11th example of the pain management support information on the doctor side.
Fig. 28 is a diagram illustrating a 12th example of the pain management support information on the doctor side.
Fig. 29 is a diagram illustrating an exemplary processing procedure by the pain management support device.

### Description of Embodiments

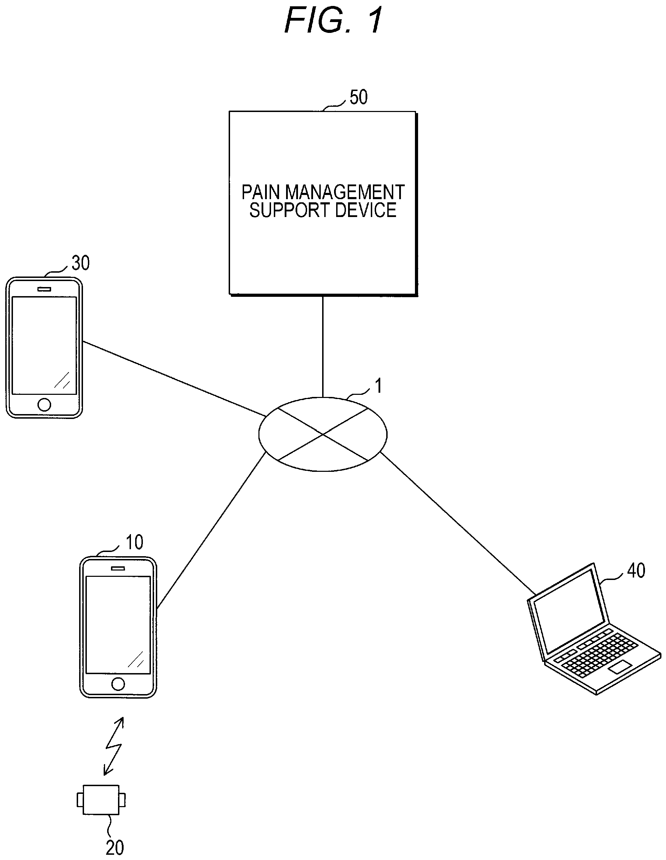

Hereinafter, an embodiment of the present invention will be described. Fig. 1 is a diagram illustrating an exemplary configuration of a pain management support system according to the present embodiment. The pain management support system includes a pain management support device 50. The pain management support system may include a patient terminal device 10, a device 20, a support person terminal device 30, and a doctor terminal device 40. The patient terminal device 10, the support person terminal device 30, and the doctor terminal device 40 are connected to the pain management support device 50 via a communication network 1. The device 20 may be connected to the patient terminal device 10 by wire or wirelessly, and may be connected to the pain management support device 50 via the communication network 1. Note that the device 20 is not essential, and for example, and an input may be made only from the patient terminal device 10.

The patient terminal device 10 is a terminal device carried or held by a patient, and the support person terminal device 30 is a terminal device carried or held by a support person, such as a family member or a caregiver for the patient. The patient terminal device 10 and the support person terminal device 30 may be configured by a smartphone, a tablet, a personal computer, or the like including a display panel, an operation panel, a microphone, a speaker, or the like. An application for using the pain management support system is installed in the patient terminal device 10 and the support person terminal device 30. The patient is assumed to be, for example, a patient suffering from cancer or the like who regularly visits a medical institution while recuperating at home, but is not limited to such a patient. Note that the application may be a web application, or may be a download application.

The device 20 is a measuring instrument that measures vital data of the patient, which may be a wearable terminal, or may be a stationary type. The device 20 may measure vital data such as a body temperature, a heart rate, brain waves, electrodermal activity (EDA), and the like. Note that the vital data is not limited thereto, and may include any data assumed to be related to pain caused by a disease of the patient. For example, the vital data may include a blood pressure, a pulse pressure, a pulse wave, a blood glucose level, body weight, the number of steps, an amount of activity, and the like.

The doctor terminal device 40 is a terminal device used by a doctor in a medical institution or the like. The doctor terminal device 40 may be configured by a personal computer, a tablet, or the like including a display panel, an operation panel, or the like. The doctor terminal device 40 may be used by a medical worker, such as a public health nurse or a nurse, under the guidance of a doctor. An application (e.g., web application, etc.) for using the pain management support system is installed in the doctor terminal device 40.

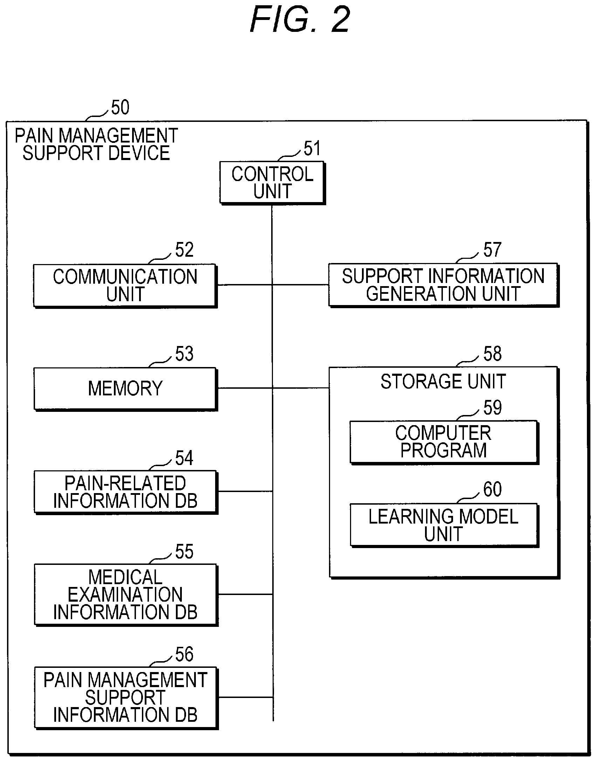

Fig. 2 is a diagram illustrating an exemplary configuration of the pain management support device 50. The pain management support device 50 includes a control unit 51 that takes overall control of the device, a communication unit 52, a memory 53, a pain-related information database (DB) 54, a medical examination information DB 55, a pain management support information DB 56, a support information generation unit (generation unit) 57, and a storage unit 58. The pain management support device 50 may be configured by a plurality of devices by distributing processing functions. The pain-related information DB 54, the medical examination information DB 55, and the pain management support information DB 56 may be incorporated in the pain management support device 50, or may be incorporated in an external data server. Furthermore, the storage unit 58 may be incorporated in an external data server.

The storage unit 58 may be configured by, for example, a hard disk, a semiconductor memory, or the like, and stores a computer program 59 (program product), a learning model unit 60, and required information. The computer program 59 may be downloaded from an external device via the communication unit 52 and stored in the storage unit 58. Furthermore, the computer program 59 recorded in a recording medium (e.g., optically-readable disk storage medium such as compact disc read only memory (CD-ROM), etc.) may be read by a recording medium reader and stored in the storage unit 58, or the computer program 59 may be read by the recording medium reader and loaded into the memory 53.

The control unit 51 is configured by incorporating a required number of central processing units (CPUs), micro-processing units (MPUs), graphics processing units (GPUs), and the like. The control unit 51 may execute processing defined by the computer program 59. That is, the processing executed by the control unit 51 corresponds to processing executed in accordance with the computer program 59. The control unit 51 may implement the function of the support information generation unit 57 by executing the computer program 59. The support information generation unit 57 may be configured by hardware, software, or a combination of hardware and software. The control unit 51 and the support information generation unit 57 may also perform processing using the learning model unit 60. The learning model unit 60 may not be an essential component.

The communication unit 52 includes a communication module, and may exchange required information with the patient terminal device 10, the support person terminal device 30, and the doctor terminal device 40 via the communication network 1. Furthermore, the communication unit 52 may exchange required information with the device 20.

The memory 53 may be configured by a semiconductor memory, such as a static random access memory (SRAM), a dynamic random access memory (DRAM), or a flash memory. The computer program 59 may be loaded into the memory 53 so that the control unit 51 is enabled to execute the computer program 59.

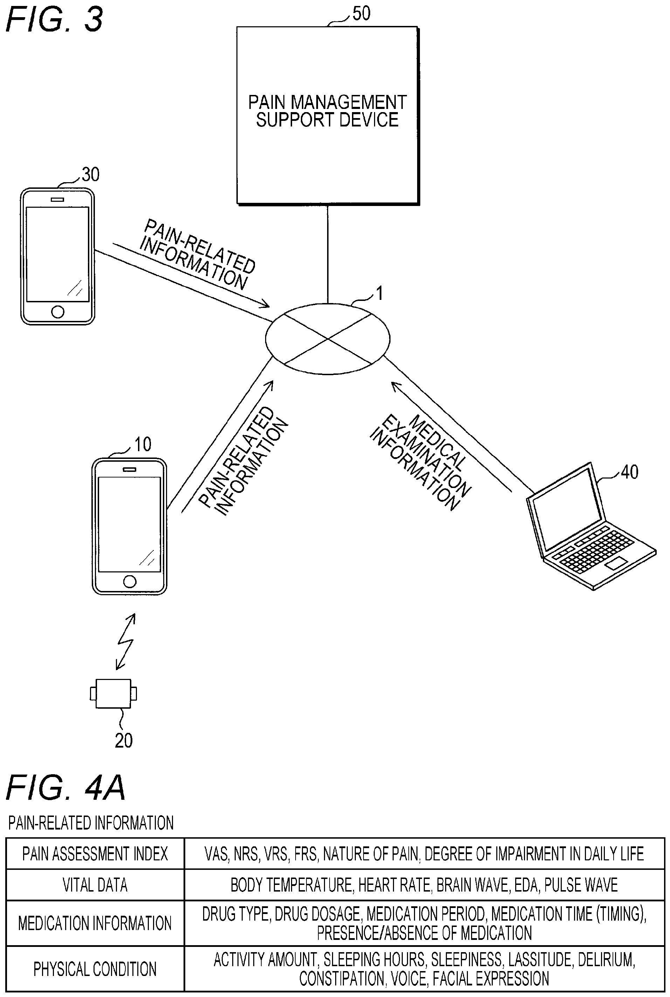

Fig. 3 is a diagram illustrating information collection by the pain management support device 50. The pain management support device 50 obtains pain-related information, which is related to pain caused by a disease of the patient, from the patient terminal device 10, the device 20, and the support person terminal device 30 via the communication unit 52. The pain-related information is repeatedly transmitted from the patient terminal device 10, the device 20, and the support person terminal device 30 as appropriate. For example, the pain-related information may be transmitted once within a period from the previous doctor visit to the next doctor visit by the patient, or may be transmitted a plurality of times depending on the length of the period. The obtained pain-related information is classified for each patient and each acquisition time point, and is stored in the pain-related information DB 54.

Furthermore, the pain management support device 50 obtains medical examination information, which is related to a medical examination of the patient, from the doctor terminal device 40 via the communication unit 52. The obtained medical examination information is classified for each patient and each medical examination time point, and is stored in the medical examination information DB 55.

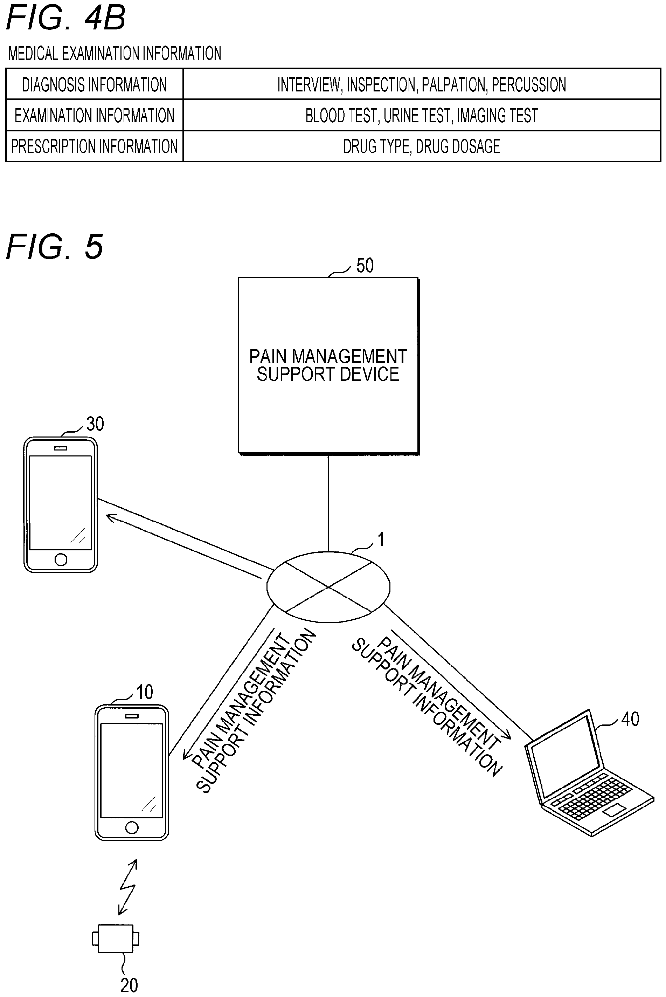

Fig. 4A is a diagram illustrating an example of the pain-related information, and Fig. 4B is a diagram illustrating an example of the medical examination information. As illustrated in Fig. 4A, the pain-related information may be classified into a pain assessment index (assessment index for assessing pain), vital data, medication information, and a physical condition. The pain-related information transmitted from the patient terminal device 10 and the device 20 includes at least one of the pain assessment index, the vital data, the medication information, and the physical condition. Note that the classification is an example, and is not limited to the example of Fig. 4A.

The pain assessment index includes a subjective assessment such as a visual analog scale (VAS), a numeric rating scale (NRS), a verbal rating scale (VRS), a face rating scale (FRS), a nature of pain, and a degree of impairment in daily life, or an objective assessment made by a medical worker or the like. The VAS, the NRS, the VRS, and the FRS are all indexes representing intensity of pain. The VAS is a visual scale for indicating a current degree of pain, and is presented to the patient to receive an answer. The NRS is a stepwise scale for indicating a current degree of pain by dividing the pain into 11 stages of 0 to 10. The VRS is a scale for indicating pain in four stages. The FRS is a facial expression scale for representing intensity of pain with facial expression. The nature of pain may distinguish the pain or symptom that has been felt, and for example, a degree of the pain is requested to be answered for pain types such as "throbbing pain", "acute pain", "pain felt when being touched", "tingling", and "itching". The degree of impairment in daily life is to evaluate a degree of influence exerted by the pain in daily life, and for example, answers are requested for questions such as whether the pain is felt in a resting state, whether the pain is felt in a moving state, whether the patient can go out, whether the patient cannot sleep due to the pain, and the like.

The vital data includes those that change in response to pain, such as a body temperature, a heart rate, brain waves, EDA, and pulse waves. Note that a blood pressure, a pulse pressure, a blood glucose level, body weight, the number of steps, an amount of activity, and the like may be included.

The medication information includes a drug type, a drug dosage, a medication period, medication time (timing), taking or not taking medication, and the like. The medication information may include not only a medicine currently taken but also a medicine previously taken. In addition, a medication period may be included.

The physical condition includes an amount of activity, sleeping hours, sleepiness, lassitude, a delirium, constipation, voice, facial expression, and the like. Items that may evaluate whether or not a disorder is caused by the pain, medicine, disease condition, or the like may be included.

By obtaining the pain-related information as described above from the patient terminal device 10 and the device 20, the patient is enabled to exactly report the pain and symptoms felt by him/herself in the period from the previous examination to the next examination.

As illustrated in Fig. 4B, the medical examination information may be classified into diagnosis information, examination information, and prescription information. The medical examination information transmitted from the doctor terminal device 40 includes at least one of the diagnosis information, the examination information, and the prescription information. Note that the classification is an example, and is not limited to the example of Fig. 4B.

The diagnosis information includes findings based on an interview, inspection, palpation, percussion, and the like at the time of the examination, an electronic medical chart, and the like. The examination information includes a blood test, a urine test, an imaging test, and the like. The prescription information includes a drug type and a drug dosage prescribed by the doctor. In addition, a medication period may be included.

Fig. 5 is a diagram illustrating provision of pain management support information by the pain management support device 50. The pain management support device 50 (control unit 51) reads pain-related information from the pain-related information DB 54 for a target patient, reads medical examination information from the medical examination information DB 55, reads pain management support information generated in the past from the pain management support information DB 56, and generates pain management support information associated with the patient on the basis of the pieces of read information. The pain management support device 50 divides the generated pain management support information into information to be provided to the patient side and information to be provided to the doctor side, and transmits the information to the patient terminal device 10 and the doctor terminal device 40 via the communication unit 52. The transmission timing may be determined at necessary timing and at a necessary frequency. Note that, among the pieces of pain management support information to be provided to the patient side, information preferable to be provided to a support person, such as a family member or a caregiver for the patient, may be transmitted to the support person terminal device 30. Furthermore, among the pieces of pain management support information to be provided to the patient side, information preferable to be provided only to the support person without being provided to the patient may be transmitted to the support person terminal device 30 instead of being transmitted to the patient terminal device 10.

As described above, by transmitting the pain management support information to the patient side, it becomes possible to intervene in the treatment of the patient at necessary timing and at a necessary frequency, which leads to improvement of QOL of the patient.

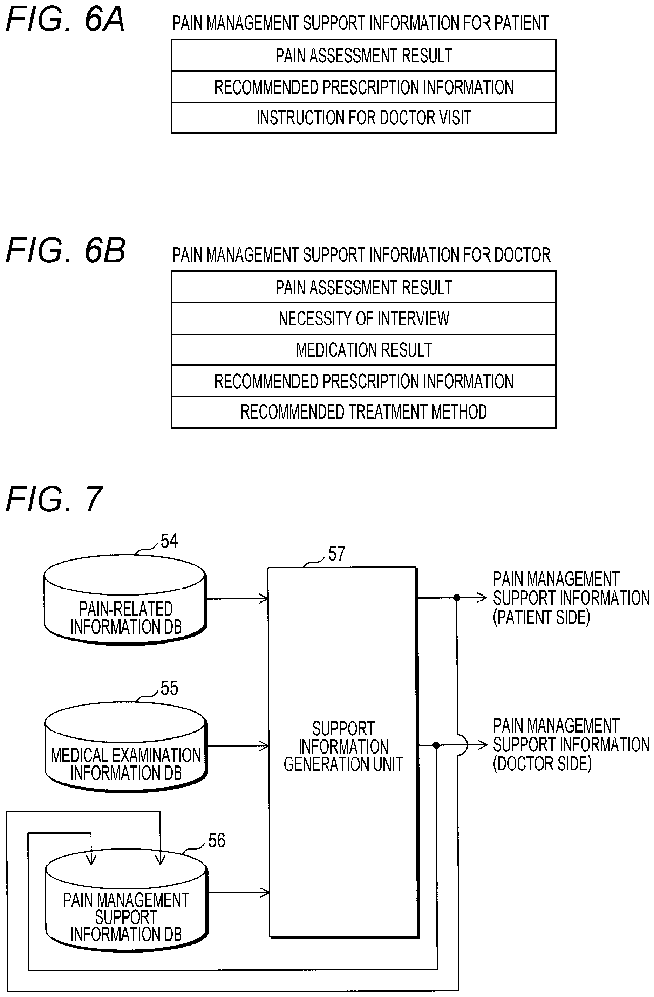

Fig. 6A is a diagram illustrating an example of the pain management support information for the patient. Fig. 6B is a diagram illustrating an example of the pain management support information for the doctor. As illustrated in Fig. 6A, the pain management support information for the patient (provided to the patient side) includes, for example, a pain assessment result, recommended prescription information, an instruction for a doctor visit, and the like. In addition, the pain management support information for the patient may include a part of the pain-related information of the patient obtained from the patient terminal device 10 and the device 20.

As illustrated in Fig. 6B, the pain management support information for the doctor (provided to the doctor side) includes, for example, a pain assessment result, necessity of an interview, a medication result, recommended prescription information, a recommended treatment method, and the like. In addition, the pain management support information for the doctor may include a part of the pain-related information of the patient obtained from the patient terminal device 10 and the device 20.

Fig. 7 is a diagram illustrating a method of generating the pain management support information. The support information generation unit 57 reads the pain-related information of the target patient from the pain-related information DB 54, reads the medical examination information of the patient from the medical examination information DB 55, and generates pain management support information for the patient on the basis of the read pain-related information and the medical examination information. At the time of generating the pain management support information, the support information generation unit 57 generates the pain management support information to be provided to the patient side and the pain management support information to be provided to the doctor side separately. The support information generation unit 57 stores the generated pain management support information in the pain management support information DB 56. Furthermore, the support information generation unit 57 may read the past pain management support information associated with the target patient from the pain management support information DB 56 in addition to the pain-related information and the medical examination information, and may generate the pain management support information associated with the patient on the basis of the read pain management support information.

For example, the support information generation unit 57 may perform rule-based generation of the pain management support information using a table indicating a correspondence relationship between the pain management support information and the pain-related information and the medical examination information. The table may indicate a correspondence relationship between the pain management support information and the pain-related information, the medical examination information, and the past pain management support information.

Furthermore, the support information generation unit 57 may use a learning model generated by machine learning in the learning model unit 60. The learning model is a model using an algorithm obtained by machine learning, and for example, a support vector machine (SVM), a decision tree, a random forest, adaptive boosting (AdaBoost), a neural network, or the like may be used.

Next, a specific example of the pain management support information will be described. In the following example, a voice input may be used when an input by the patient or the like is required.

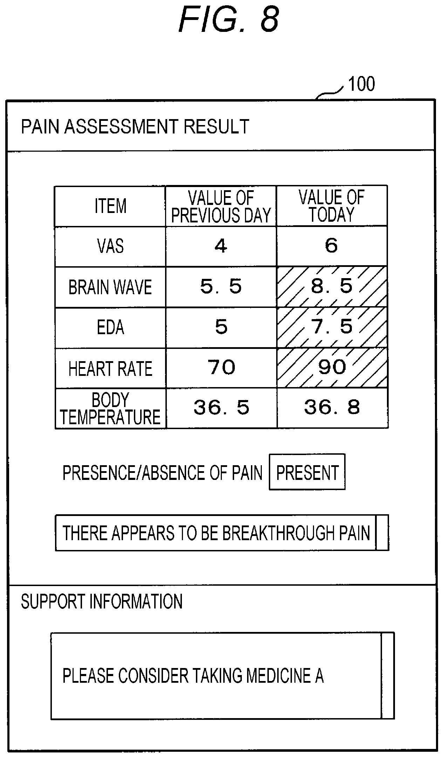

Fig. 8 is a diagram illustrating a first example of pain management support information 100 on the patient side. The pain management support information on the patient side is displayed on the display panel of the patient terminal device 10 (or the support person terminal device 30), and the patient may perform an input operation or a selection operation depending on the displayed contents. The pain management support information 100 includes a pain assessment result and support information. In the pain assessment result, a value of the previous day and a value of today are displayed in a comparable manner for each item of the VAS, the brain waves, the EDA, the heart rate, and the body temperature included in the pain-related information of the patient. Values converted to pain indexes are displayed for the VAS, the brain waves, and the EDA. In addition, presence or absence of pain and a type or pattern of the pain ("there appears to be breakthrough pain" in the example of the drawing) are displayed in the pain assessment result. The determination that "there appears to be breakthrough pain" is based on, for example, the fact that the values of the brain waves, the EDA, and the heart rate are larger today than the previous day. The support information for the patient includes, for example, recommended prescription information such as "please consider taking a medicine A". Note that the example of Fig. 8 is an example, and the display is not limited to the example of Fig. 8. For example, the values of the individual items may be shown in a graph instead of the table format. Note that there may be a case where it is not desirable to show the values converted to the pain indexes to the patient, and in such a case, the values converted to the pain indexes may not be displayed.

As described above, the control unit 51 may output, to the patient terminal device 10, the pain assessment result indicating the presence or absence of pain of the patient analyzed on the basis of the pain-related information, and may output the recommended prescription information to the patient terminal device 10 if there is pain.

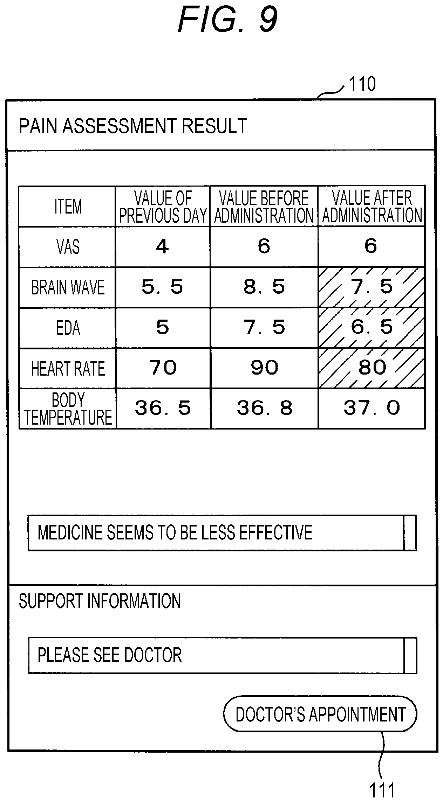

Fig. 9 is a diagram illustrating a second example of pain management support information 110 on the patient side. The pain management support information 110 includes a pain assessment result and support information. In the pain assessment result, a value of the previous day, a value before administration of a medicine, and a value after the administration of the medicine are displayed in a comparable manner for each item of the VAS, the brain waves, the EDA, the heart rate, and the body temperature included in the pain-related information of the patient. While the values of the brain waves, the EDA, and the heart rate after the administration are smaller than those before the administration, they are still larger than those of the previous day. On the basis of those changes, an evaluation result "the medicine seems to be less effective" is determined and displayed. The support information for the patient includes, for example, an instruction for a doctor visit such as "please see a doctor". When the patient operates a "doctor's appointment" icon 111, a screen for making an appointment to see a doctor is displayed, and an appointment may be made. Note that, although illustration is omitted, when the evaluation value abnormally decreases due to an excessive effect of the medicine or the like, such a numerical value is displayed, and the support information may be displayed as "please stop taking the medicine once".

As described above, the control unit 51 may output, to the patient terminal device 10, the pain assessment result indicating the administration result before and after the medicine administration based on the recommended prescription information, and may output the instruction for a doctor visit to the patient terminal device 10 on the basis of the administration result.

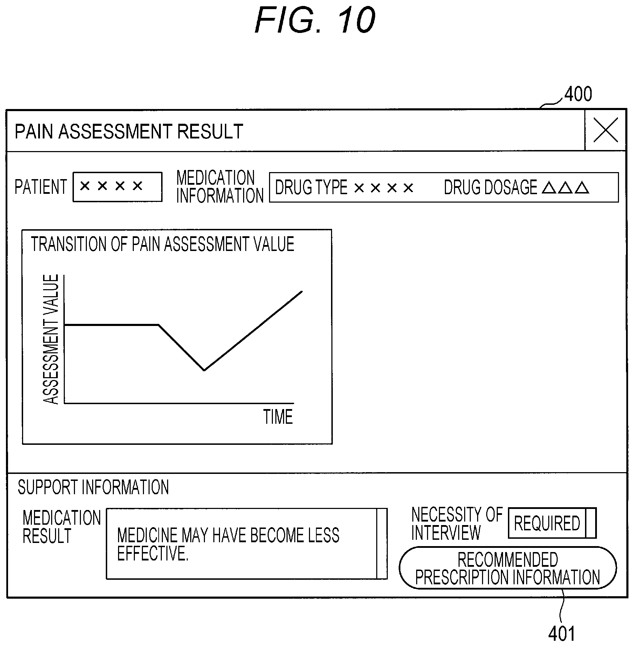

Fig. 10 is a diagram illustrating a first example of pain management support information 400 on the doctor side. The pain management support information on the doctor side is displayed on the display panel of the doctor terminal device 40, and the doctor may perform an input operation or a selection operation depending on the displayed contents. The pain management support information 400 includes a pain assessment result and support information. In the pain assessment result, patient information (patient ID, patient name, etc.), medication information (drug type, drug dosage, medication period, etc.), and transition of a pain assessment value are displayed. The pain assessment value may be converted on the basis of the pain assessment index included in the pain-related information and required information in the vital data. In the example of Fig. 10, while the pain assessment value temporarily decreases due to the administration of the medicine, it increases again. A medication effect and necessity of an interview are displayed in the support information. The medication effect may be selected on the basis of analysis of the transition of the pain assessment value. In the example of Fig. 10, it is displayed as "the medicine may have become less effective". In such a case, the doctor considers that the patient needs to be examined, and thus the necessity of the interview is "required". When the doctor operates a "recommended prescription information" icon 401, the recommended prescription information to be described later may be displayed.

As described above, the control unit 51 may output, to the doctor terminal device 40, a temporal change of the pain assessment result indicating an effect of an analgesic on the patient who is taking the analgesic, and may output the necessity of an interview to the doctor terminal device 40 on the basis of the effect of the analgesic.

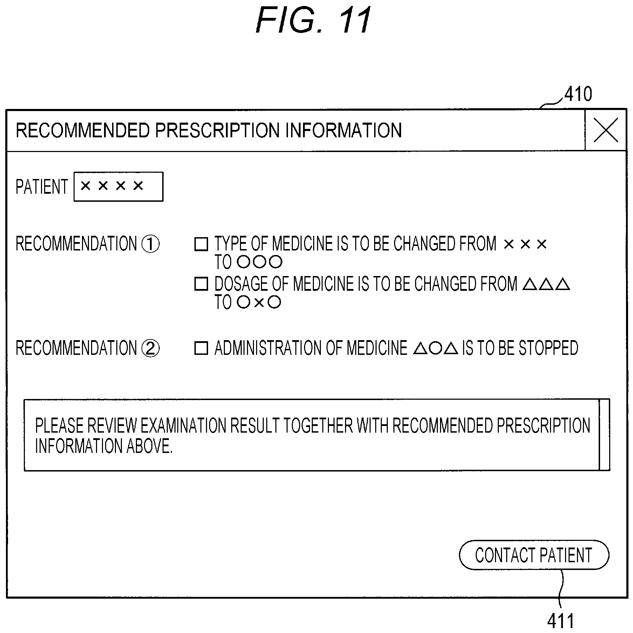

Fig. 11 is a diagram illustrating a second example of pain management support information 410 on the doctor side. The pain management support information 410 includes recommended prescription information. In the recommended prescription information, recommended contents are displayed together with the patient information. In the example of Fig. 11, a change in the drug type and a change in the drug dosage are displayed as recommendation 1. As recommendation 2, it is recommended that administration of a specific medicine is stopped. Furthermore, in addition to the recommended contents, guidance to the doctor may be displayed such as "please review the examination result together with the recommended prescription information above". By operating a "contact patient" icon 411, the doctor may recommend that an appointment to see the doctor is to be scheduled on a date and time before the next scheduled appointment date, or may make notification that administration of a specific medicine is to be stopped.

As described above, the control unit 51 may output the recommendation of the medication change to the doctor terminal device 40 on the basis of the effect of the analgesic.

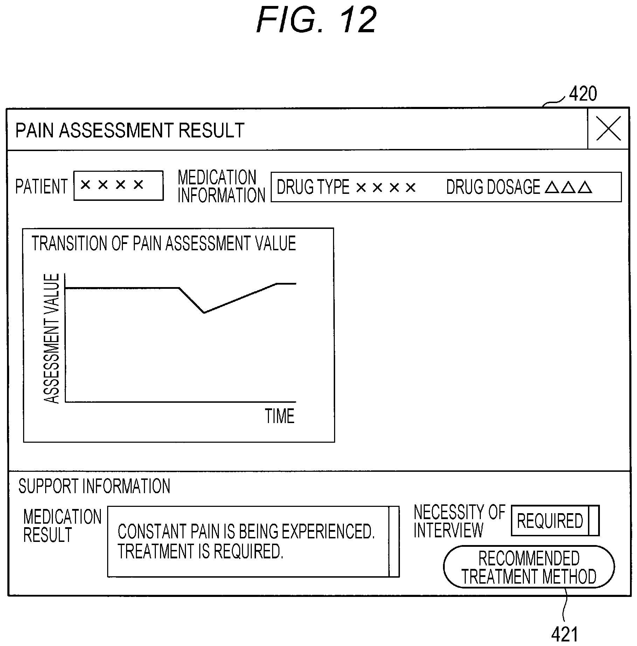

Fig. 12 is a diagram illustrating a third example of pain management support information 420 on the doctor side. The pain management support information 420 includes a pain assessment result and support information. In the pain assessment result, in a similar manner to the case of the first example exemplified in Fig. 10, the patient information (patient ID, patient name, etc.), the medication information (drug type, drug dosage, medication period, etc.), and the transition of the pain assessment value are displayed. As compared with the first example, in the third example, the pain assessment value has transitioned at a higher value, and although it has temporarily decreased due to the administration of the medicine, it has transitioned at a higher value again. A medication effect and necessity of an interview are displayed in the support information. The medication effect may be selected on the basis of analysis of the transition of the pain assessment value. In the example of Fig. 12, it is displayed as "Constant pain is being experienced. Treatment is required". In such a case, the doctor considers that the patient needs to be examined, and thus the necessity of the interview is "required". When the doctor operates a "recommended treatment method" icon 421, the recommended treatment method to be described later may be displayed.

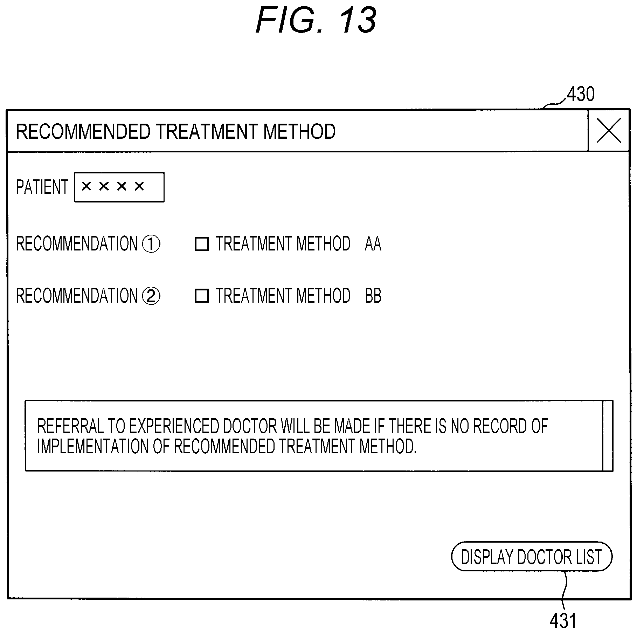

Fig. 13 is a diagram illustrating a fourth example of pain management support information 430 on the doctor side. The pain management support information 430 includes a recommended treatment method. In the recommended treatment method, recommended contents are displayed together with the patient information. In the example of Fig. 13, a treatment method AA is displayed as recommendation 1, and a treatment method BB is displayed as recommendation 2. Furthermore, in addition to the recommended contents, it is displayed as "referral to an experienced doctor will be made if there is no record of implementation of the recommended treatment method". When the doctor operates a "display doctor list" icon 431, referral information regarding doctors experienced in the recommended treatment method is displayed in a list format. The referral information may include, for example, information such as a medical institution name, a doctor name, an experienced treatment method, a contact, and the like. Furthermore, a "contact" icon or the like may be operated to contact a required doctor.

As described above, the control unit 51 may output, to the doctor terminal device 40, the temporal change of the pain assessment result indicating the effect of the analgesic on the patient who is taking the analgesic, and may output, to the doctor terminal device 40, the recommended treatment method or the referral information regarding doctors experienced in the recommended treatment method on the basis of the effect of the analgesic.

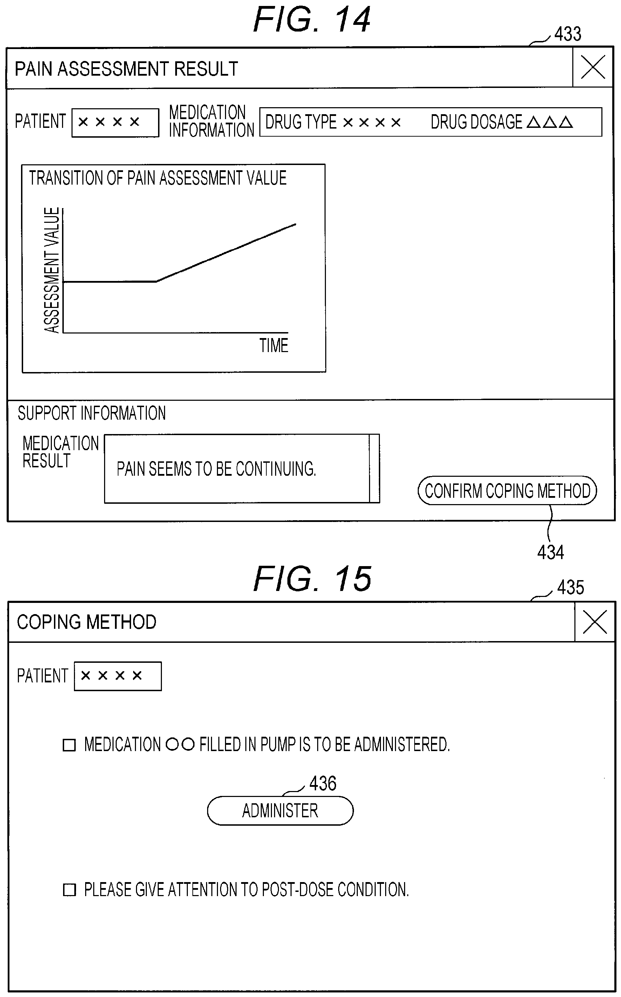

Fig. 14 is a diagram illustrating a fifth example of pain management support information 433 on the doctor side. The pain management support information 433 includes a pain assessment result and support information. In the pain assessment result, in a similar manner to the case of the first example exemplified in Fig. 10, the patient information (patient ID, patient name, etc.), the medication information (drug type, drug dosage, medication period, etc.), and the transition of the pain assessment value are displayed. In the case of the fifth example, the pain assessment value increases from a certain time point. The medication effect is displayed in the support information. The medication effect may be selected on the basis of analysis of the transition of the pain assessment value. In the example of Fig. 14, it is displayed as "pain seems to be continuing". In such a case, a coping method may be presented to the doctor as the pain management support information. When the doctor operates a "confirm coping method" icon 434, the coping method to be described later may be displayed.

Fig. 15 is a diagram illustrating a sixth example of pain management support information 435 on the doctor side. The pain management support information 435 includes a coping method. In the example of Fig. 15, it is displayed as "the medication OO filled in a pump is to be administered", and an "administer" icon 436 is displayed. Examples of the pump include a syringe driver. Furthermore, in the coping method, an alert to the doctor is displayed such as "please give attention to post-dose conditions". When the doctor operates the "administer" icon 436, the medication connected to the pump may be administered to the patient by the operation of the doctor. Furthermore, an instruction may be transmitted to a medical worker, a caregiver, or the like to activate the pump at a distant place such as at home. As a result, the remote operation of the pump may not be required.

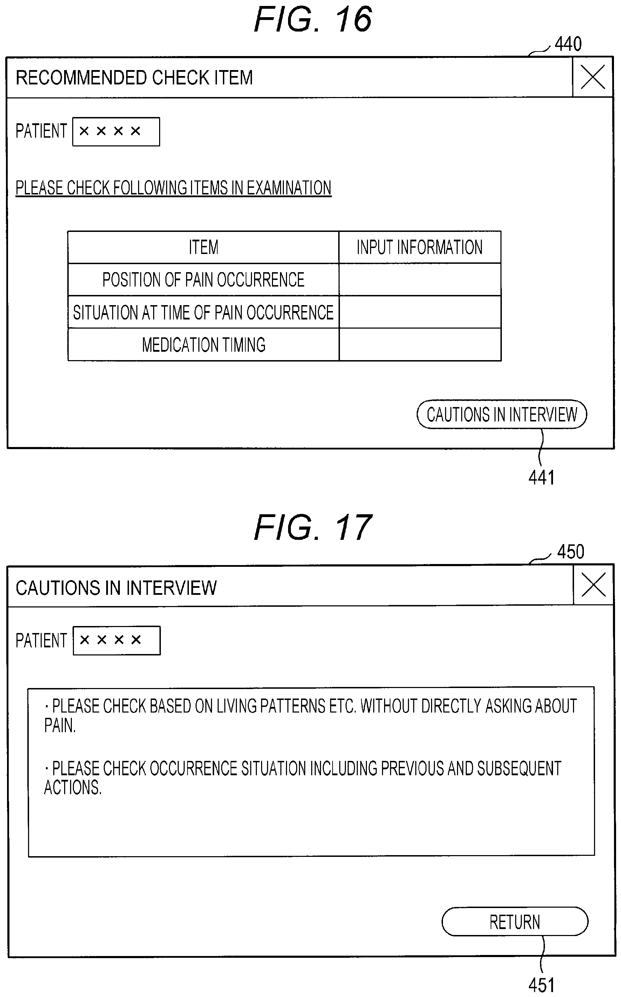

Fig. 16 is a diagram illustrating a seventh example of pain management support information 440 on the doctor side. The pain management support information 440 includes recommended check items. The recommended check items indicate information for recommending check items that the doctor needs to confirm with the patient at the next examination. That is, the control unit 51 may output, to the doctor terminal device 40, the check items needed to select recommended prescription information or a recommended treatment method at the time of the examination. At the time of generating the pain management support information, the support information generation unit 57 outputs, as recommended check items, information required for analysis to the doctor terminal device 40 for display, and obtains the recommended check items from the doctor terminal device 40. As a result, the support information generation unit 57 is enabled to generate the pain management support information.

In Fig. 16, a position of pain occurrence, a situation at the time of the pain occurrence, and medication timing are displayed as items of the recommended check items. The doctor may confirm each item with the patient at the next medical examination and input the input information. When the doctor operates a "cautions in interview" icon 441, cautions in the interview to be described later are displayed.

Fig. 17 is a diagram illustrating an eighth example of pain management support information 450 on the doctor side. The pain management support information 450 includes cautions in an interview. The cautions in the interview are displayed as, for example, "please check based on living patterns etc. without directly asking about pain", and "please check the occurrence situation including previous and subsequent actions". As a result, the doctor is enabled to treat the patient in a manner of having a skill or experience of extracting appropriate information from the patient, and to achieve appropriate communication with the patient. When a "return" icon 451 is operated, the screen returns to the display screen of Fig. 16.

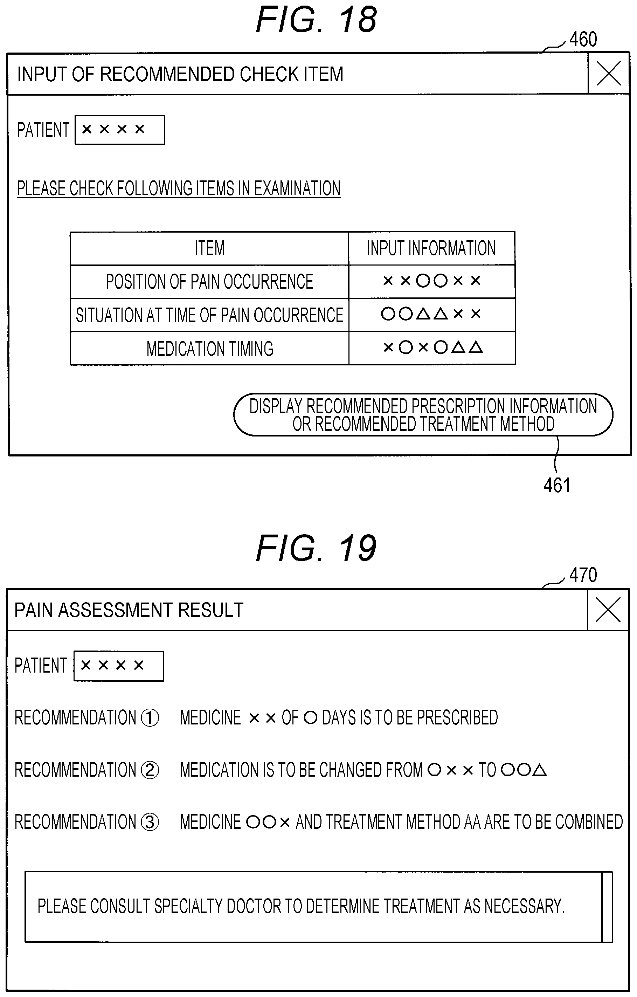

Fig. 18 is a diagram illustrating a ninth example of pain management support information 460 on the doctor side. The pain management support information 460 indicates a state at the time of inputting the recommended check items. The doctor may input the input information for each item obtained in the interview at the time of the medical examination. With the recommended check items transmitted from the doctor terminal device 40 to the pain management support device 50, the pain management support device 50 (control unit 51) is enabled to generate the pain management support information including the recommended prescription information or the recommended treatment method. When the doctor operates a "display recommended prescription information or recommended treatment method" icon 461, recommendation information to be described later is displayed.

Fig. 19 is a diagram illustrating a tenth example of pain management support information 470 on the doctor side. The pain management support information 470 includes recommendation information. In the recommendation information, a prescription of a medicine is displayed as recommendation 1, a change of a medicine is displayed as recommendation 2, and a combination of a medicine and a treatment method is displayed as recommendation 3. Furthermore, as illustrated in Fig. 19, a proposal such as "please consult a specialty doctor to determine treatment as necessary" may be displayed.

As described above, the control unit 51 may receive the input information for the check items and output the recommended prescription information or the recommended treatment method to the doctor terminal device 40 on the basis of the received input information.

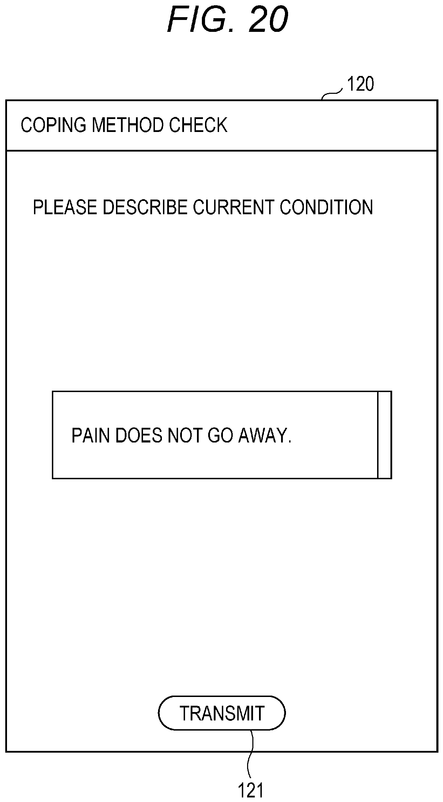

Fig. 20 is a diagram illustrating a third example of pain management support information 120 on the patient side. The pain management support information 120 includes information for checking a coping method. The patient may operate the application of the patient terminal device 10 to display a coping method confirmation screen. A description such as "please describe a current condition" is displayed in the pain management support information 120. For example, the patient may input the current state of pain or symptom such as "pain does not go away". When the patient operates a "transmit" icon 121, the input information is transmitted to the pain management support device 50. Note that a plurality of formats indicating current pain states and symptoms may be prepared, and the plurality of formats may be displayed to cause the patient to make a selection such that the selected format is transmitted to the pain management support device 50.

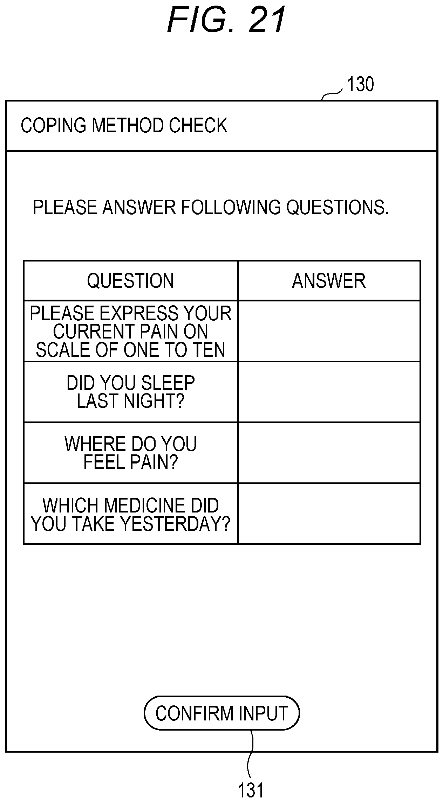

Fig. 21 is a diagram illustrating a fourth example of pain management support information 130 on the patient side. The pain management support information 130 includes information for checking a coping method. When the pain management support device 50 (control unit 51) obtains the input information indicating the current state of pain or symptom of the patient, it transmits questions for determining the current condition of the patient in more detail to the patient terminal device 10. The questions include, for example, "please express your current pain on a scale of one to ten", "did you sleep last night?", "where do you feel pain?", "which medicine did you take yesterday?", and the like. The patient inputs answers to the questions, and operates a "confirm input" icon 131, whereby the answers to the questions are transmitted to the pain management support device 50.

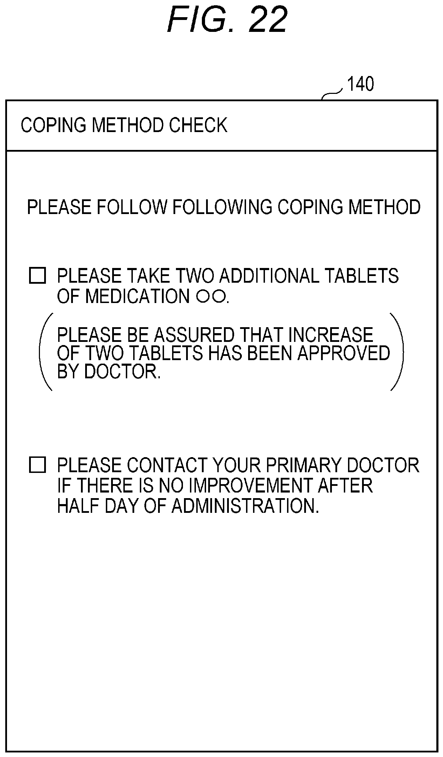

Fig. 22 is a diagram illustrating a fifth example of pain management support information 140 on the patient side. Upon reception of the answers to the questions, the pain management support device 50 (control unit 51) may analyze the answers to generate a coping method, and may output the generated coping method to the patient terminal device 10. For example, as illustrated in Fig. 22, it is displayed as "Please take two additional tablets of the medication OO. (Please be assured that the increase of two tablets has been approved by the doctor)", and "Please contact your primary doctor if there is no improvement after half a day of the administration". Note that an audio output may be made instead of displaying characters.

As described above, the control unit 51 may output the questions for evaluating the current state of pain of the patient to the patient terminal device 10 or the support person terminal device 30, and may receive the input of the answers to the questions to output the coping method to the patient terminal device 10 on the basis of the received answers.

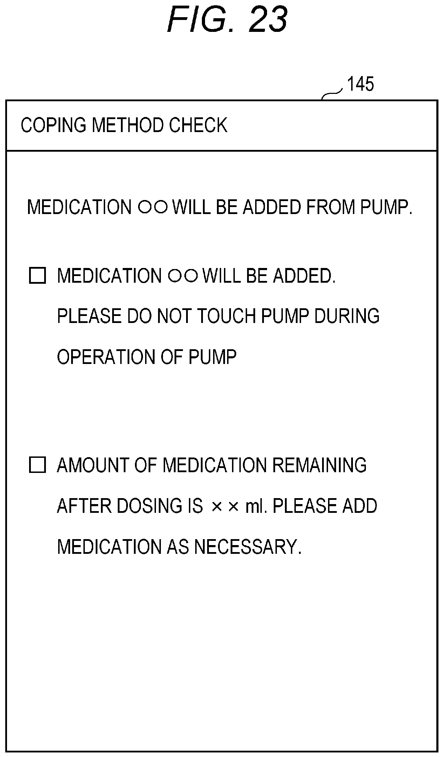

Fig. 23 is a diagram illustrating a sixth example of pain management support information 145 on the patient side. Upon reception of the answers to the questions, the pain management support device 50 (control unit 51) may analyze the answers to generate a coping method, and may output the generated coping method to the patient terminal device 10. For example, as illustrated in Fig. 23, it is displayed as "the medication OO will be added from the pump". Dosing of a medicine may be carried out by an operation made by the patient side. In this case, an alert to the patient may be displayed such as "please do not touch the pump during the operation of the pump". Furthermore, an alert to the patient may be displayed such as "The amount of the medication remaining after the dosing is XX ml of the medication. Please add the medication as necessary" as appropriate. Note that an audio output may be made instead of displaying characters.

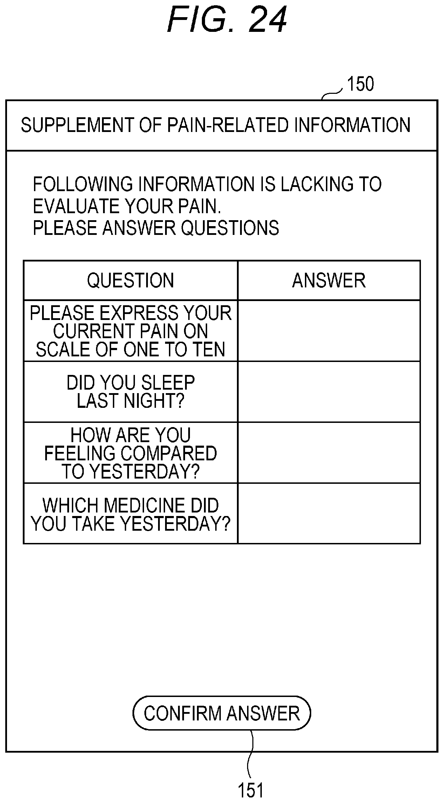

Fig. 24 is a diagram illustrating a seventh example of pain management support information 150 on the patient side. The pain management support information 150 includes information for supplementing the pain-related information. When there is a lack of information for evaluating the pain or symptom of the patient, the control unit 51 may output a question for obtaining the lacking information to the patient terminal device 10. For example, it may be displayed as "The following information is lacking for evaluation of your pain. Please answer the questions.", and a questionnaire may be displayed. In the example of Fig. 24, questions such as "please express your current pain on a scale of one to ten", "did you sleep last night?", "how are you feeling compared to yesterday?", and "which medicine did you take yesterday?", and answer columns for the questions are displayed. The patient inputs answers in the answer columns, and operates a "confirm answer" icon 151, whereby the answers to the questions are transmitted to the pain management support device 50.

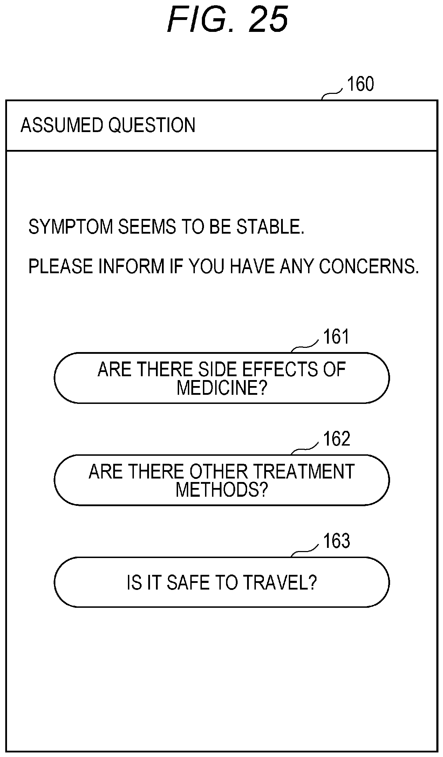

Fig. 25 is a diagram illustrating an eighth example of pain management support information 160 on the patient side. When the pain management support device 50 (control unit 51) obtains the answers to the questions exemplified in Fig. 24, it evaluates the pain or symptom of the patient, and outputs an evaluation result to the patient terminal device 10. In the example of Fig. 25, it is displayed as "the symptom seems to be stable" as the evaluation result. Furthermore, the pain management support device 50 (control unit 51) may display assumed questions related to the pain or symptom of the patient together with the evaluation result. The assumed questions are questions that the patient may have or questions of interest to the patient based on the current pain or symptom of the patient, and include information that the patient is likely to ask. In the example of Fig. 25, assumed questions such as "are there side effects of the medicine?", "are there other treatment methods?", "is it safe to travel?" are displayed, and the patient may operate individual icons 161, 162, and 163 to obtain answers to the assumed questions. Hereinafter, a case will be described in which the icon 163 of "is it safe to travel?" is operated.

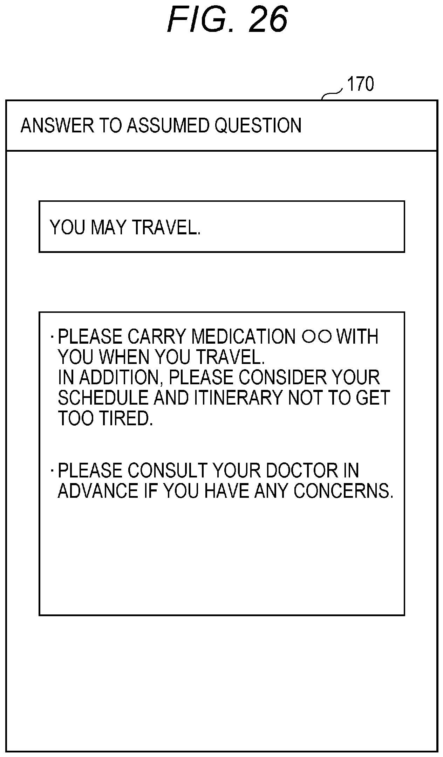

Fig. 26 is a diagram illustrating a ninth example of pain management support information 170 on the patient side. The pain management support information 170 includes an answer to the assumed question. In the example of Fig. 26, the answer is displayed as "You may travel", "Please carry the medication OO with you when you travel. In addition, please consider your schedule and itinerary not to get too tired", and "Please consult your doctor in advance if you have any concerns". Note that an audio output may be made instead of displaying characters.

As described above, the control unit 51 may output the plurality of assumed questions related to the pain or symptom of the patient to the patient terminal device 10, and may output the answer to the assumed question selected by the patient from among the plurality of assumed questions to the patient terminal device 10.

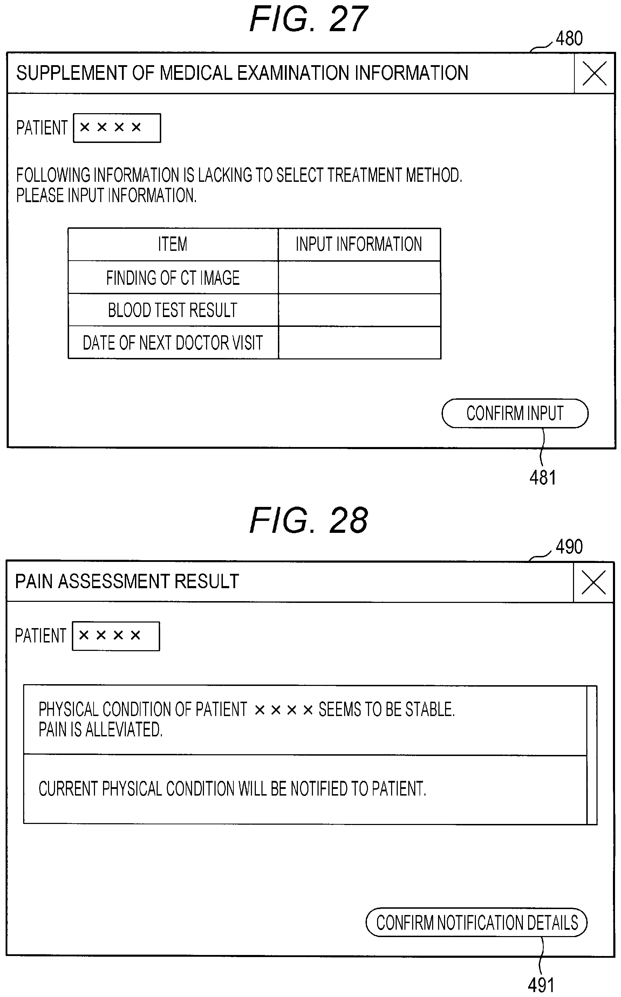

Fig. 27 is a diagram illustrating an 11th example of pain management support information 480 on the doctor side. The pain management support information 480 includes information for supplementing the medical examination information. When there is a lack of information regarding the pain or symptom of the patient or a lack of information for selecting the recommended prescription information or the recommended treatment method, the control unit 51 may output check items for obtaining the lacking information to the doctor terminal device 40. For example, it may be displayed as "The following information is lacking to select a treatment method. Please input information", and the check items may be displayed. In the example of Fig. 27, the check items such as "findings of CT images", "blood test result", and "date of next doctor visit", and entry fields for the check items are displayed. The doctor inputs the input information in the entry fields and operates a "confirm input" icon 481, whereby the input information for the check items is transmitted to the pain management support device 50.

Fig. 28 is a diagram illustrating a 12th example of pain management support information 490 on the doctor side. The pain management support information 490 includes a pain assessment result of the patient. In the example of Fig. 28, the pain-related assessment result is displayed as "The physical condition of the patient XXXX seems to be stable. The pain is alleviated." and "The current object will be notified to the patient". When the doctor operates a "confirm notification details" icon 491, the contents to be notified to the patient by the pain management support device 50 may be displayed. As a result, the doctor is enabled to confirm the details of the notification to the patient. Note that the symptom of the patient, the recommended prescription information, or the recommended treatment method may be displayed instead of the pain assessment result.

As described above, the control unit 51 may output the check items required to generate the pain management support information to the doctor terminal device 40, and may receive the input information for the check items to generate the pain management support information on the basis of the received input information.

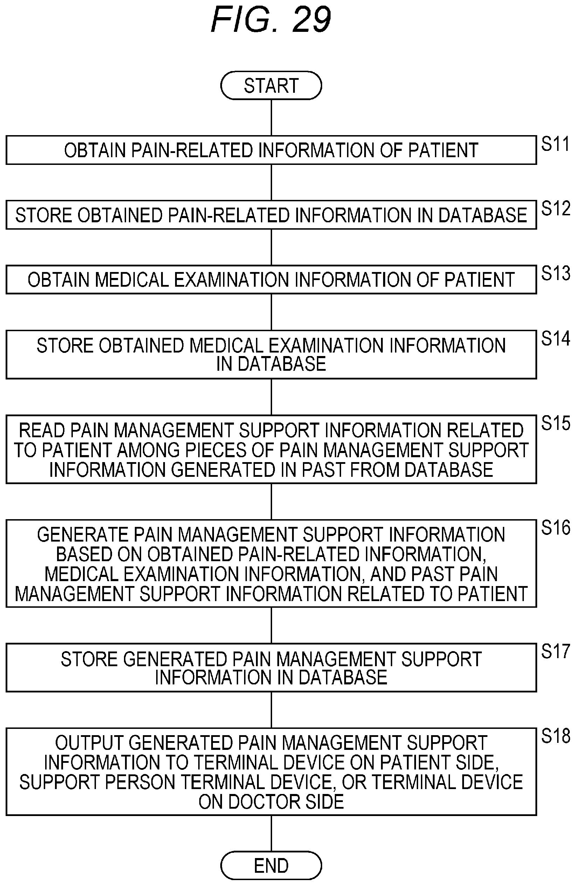

Fig. 29 is a diagram illustrating an exemplary processing procedure by the pain management support device 50. For convenience, the following descriptions will be given on the assumption that the control unit 51 takes the lead in executing the process. The control unit 51 obtains the pain-related information of the patient (S11), and stores the obtained pain-related information in the pain-related information DB 54 (database) (S12). The pain-related information may be repeatedly obtained for each patient at required timing. For example, it may be obtained one or a plurality of times within a period from the previous doctor visit to the next doctor visit.

The control unit 51 obtains the medical examination information of the patient (S13), and stores the obtained medical examination information in the medical examination information DB 55 (database) (S14). The medical examination information may be obtained not only at the time of the examination but also when it is determined to be necessary by the doctor.

The control unit 51 reads, from the pain management support information DB 56 (database), the pain management support information related to the patient among the pieces of pain management support information generated in the past (S15), and generates pain management support information on the basis of the obtained (stored) pain-related information and medical examination information and the read pain management support information (S16).

The control unit 51 stores the generated pain management support information in the pain management support information DB 56 (database) (S17), outputs the generated pain management support information to the terminal device on the patient side, the support person terminal device, or the terminal device on the doctor side (S18), and terminates the process.

As described above, according to the present embodiment, the pain and various symptoms that the patient feels may be exactly conveyed during the interview, a change of the analgesic method may be supported as appropriate, and the treatment of the patient may be intervened at a necessary frequency, whereby the pain of the patient may be alleviated to improve QOL. Furthermore, communication and mutual understanding between the patient and the doctor may be complemented to support appropriate pain management. Furthermore, information for determining a coping method (prescription information, treatment method, etc.) for alleviating the pain of the patient may be provided to support the doctor.

### Reference Signs List

- 1: Communication network
- 10: Patient terminal device
- 20: Device
- 30: Support person terminal device
- 40: Doctor terminal device
- 50: Pain management support device
- 51: Control unit
- 52: Communication unit
- 53: Memory
- 54: Pain-related information DB
- 55: Medical information DB
- 56: Pain management support information DB
- 57: Support information generation unit
- 58: Storage unit
- 59: Computer program
- 60: Learning model unit

## Claims

1. A computer program for causing a computer to execute a process comprising:
repeatedly obtaining pain-related information associated with pain of a patient at required timing;
storing the obtained pain-related information;
obtaining medical examination information associated with a medical examination of the patient;
storing the obtained medical examination information;
generating pain management support information on a basis of the pain-related information and the medical examination information; and
outputting the generated pain management support information.

2. The computer program according to claim 1, wherein
the pain-related information includes
at least one of an assessment index for assessing the pain, vital data, medication information, or a physical condition.

3. The computer program according to claim 1, wherein
the medical examination information includes
at least one of diagnosis information, examination information, or prescription information.

4. The computer program according to claim 1, wherein
the pain management support information includes
at least one of a pain assessment result, recommended prescription information, or an instruction for a doctor visit for supporting the patient.

5. The computer program according to claim 1, wherein
the pain management support information includes
at least one of a pain assessment result, necessity of an interview, a medication result, recommended prescription information, or a recommended treatment method for supporting a doctor who examines the patient.

6. The computer program according to any one of claims 1 to 5, the program causing the computer to execute the process further comprising:
outputting, to a patient terminal device or a support person terminal device, a pain assessment result indicating presence or absence of the pain of the patient on a basis of the pain-related information; and
outputting recommended prescription information to the patient terminal device or the support person terminal device when the pain is present.

7. The computer program according to claim 6, the program causing the computer to execute the process further comprising:
outputting, to the patient terminal device, a pain assessment result indicating an administration result before and after administration of a medicine based on the recommended prescription information; and
outputting an instruction for a doctor visit to the patient terminal device on a basis of the administration result.

8. The computer program according to any one of claims 1 to 5, the program causing the computer to execute the process further comprising:
outputting, to a doctor terminal device, a temporal change of a pain assessment result indicating an effect of an analgesic on the patient who is taking the analgesic; and
outputting recommendation of a medication change or necessity of an interview to the doctor terminal device on a basis of the effect of the analgesic.

9. The computer program according to any one of claims 1 to 5, the program causing the computer to execute the process further comprising:
outputting, to a doctor terminal device, a temporal change of a pain assessment result indicating an effect of an analgesic on the patient who is taking the analgesic; and
outputting, to the doctor terminal device, a coping method, a recommended treatment method, or referral information regarding a doctor experienced **in** the recommended treatment method on a basis of the effect of the analgesic.

10. The computer program according to any one of claims 1 to 5, the program causing the computer to execute the process further comprising:
outputting, to a doctor terminal device, a check item needed to select recommended prescription information or a recommended treatment method at a time of an examination;
receiving input information for the check item; and
outputting the recommended prescription information or the recommended treatment method to the doctor terminal device on a basis of the input information.

11. The computer program according to any one of claims 1 to 5, the program causing the computer to execute the process further comprising:
outputting a question for evaluating a state of the pain of the patient to a patient terminal device;
receiving an input of an answer to the question; and
outputting a coping method to the patient terminal device on a basis of the answer.

12. The computer program according to any one of claims 1 to 5, the program causing the computer to execute the process further comprising:
outputting a plurality of assumed questions related to the pain of the patient to a patient terminal device; and
outputting, to the patient terminal device, an answer to an assumed question selected by the patient from among the plurality of assumed questions.

13. The computer program according to any one of claims 1 to 5, the program causing the computer to execute the process further comprising:
outputting a check item needed to generate the pain management support information to a doctor terminal device;
receiving input information for the check item; and
generating the pain management support information on a basis of the input information.

14. A pain management support device comprising:
a first acquisition unit that repeatedly obtains pain-related information associated with pain of a patient at required timing;
a first storage unit that stores the obtained pain-related information;
a second acquisition unit that obtains medical examination information associated with a medical examination of the patient;
a second storage unit that stores the obtained medical examination information;
a generation unit that generates pain management support information on a basis of the pain-related information and the medical examination information; and
an output unit that outputs the generated pain management support information.

15. A pain management support method comprising:
repeatedly obtaining pain-related information associated with pain of a patient at required timing;
storing the obtained pain-related information;
obtaining medical examination information associated with a medical examination of the patient;
storing the obtained medical examination information;
generating pain management support information on a basis of the pain-related information and the medical examination information; and
outputting the generated pain management support information.
